# FASCICULE DE BREVET EUROPEEN

(11) **EP 0 548 435 B1**
(45) Date de publication et mention de la délivrance du brevet: **19.03.1997**
(21) Numéro de dépôt: 91403552.2
(22) Date de dépôt: 27.12.1991
(51) Int. Cl.: A61B 5/0416

(54) **Connecteur d'électrode, notamment d'électrode d'électrocardiogramme, et ensemble d'électrode muni d'un tel connecteur**
Elektrodenverbinder, besonders für Elektrokardiogrammelektrode, und zugleich Elektrode mit solch einem Verbinder
Electrode connector, particularly electrocardiogram electrode, and at the same time electrode consisting of such a connector

(43) Date de publication de la demande: 30.06.1993
(73) Titulaire: Ascher, Gilles, F-92200 Neuilly Sur Seine (FR)
(72) Inventeur: Ascher, Gilles, F-92200 Neuilly Sur Seine (FR)
(74) Mandataire: Keib, Gérard

(56) Documents cités:
- EP-A- 0 065 511
- EP-A- 0 449 800
- DE-A- 2 802 547

## Description

L'invention concerne un connecteur d'électrode, notamment d'électrode pour l'enregistrement de signaux vitaux par voie non-invasive et ainsi destinée à être appliquée sur la peau d'un patient, par exemple une électrode d'électrocardiogramme, électrode du type comportant une pastille en matériau mousse imprégné d'un gel bon conducteur de l'électricité et un pion muni d'une tête de connexion, également en matériau bon conducteur, en contact avec cette pastille. L'invention concerne également un ensemble d'électrode constitué d'une telle électrode et d'un tel connecteur.

Lors de l'enregistrement de signaux vitaux par vole non-invasive, en continu au cours d'une journée complète d'activité normale d'un patient, donc en mode ambulatoire, on utilise une électrode de ce type, comportant de plus un disque en matériau souple pouvant épouser localement la forme du corps du patient, dont une face est enduite d'un adhésif pour pouvoir être collée à l'épiderme de celui-ci, et muni d'un trou central pour pouvoir être enfilé autour de la tête d'électrode et solidarisé à la pastille en mousse imprégnée.

L'enregistrement des signaux, par exemple d'un électrocardiogramme, est réalisé au moyen de plusieurs électrodes similaires disposées en des endroits appropriés du corps, par exemple de la poitrine, du patient, de manière à enregistrer la différence de potentiel entre deux électrodes déterminées, en prenant une électrode comme potentiel de référence.

Une telle électrode est montrée dans le document EP-A-449 800 et est représentée sur la figure 1 ; on voit sur cette figure la pastille en mousse 1 imprégnée d'un gel conducteur de l'électricité destinée à être appliquée contre la peau d'un patient, accolée, à l'opposé de la peau du patient, à une embase du pion 2 ; le disque 3 muni d'un trou central de manière à pouvoir être enfilé autour du pion 1, et dont la face inférieure est enduite d'un adhésif destiné à fixer l'électrode à la peau du patient, est également en matériau mousse.

Le pion 2 comporter à l'opposé de son embase 21 accolée à la pastille 1, une tête renflée 22 destinée à l'encliquetage d'un connecteur 4.

Généralement, la tête 22 et le connecteur 4 sont réalisés de manière similaire à un bouton-pression.

A cette fin, le connecteur 4 comporte un organe de contact 41 en matériau conducteur de l'électricité destiné à venir en appui contre la tête 22 de l'électrode, cet appui étant maintenu par un organe d'encliquetage 42 tel qu'une rondelle élastique fendue ou une agraffe élastique à deux branches, ou autre, logé dans l'organe de contact 41 ; l'ensemble est lui-même logé à l'intérieur d'un capuchon de protection 43, en matériau isolant électrique. Un câble électrique à un conducteur 44 relie l'organe de contact 41 à une borne appropriée d'un appareil d'enregistrement, ici d'électrocardiogramme (non représenté). Le capuchon 43 peut éventuellement être muni d'un blindage métallique relié par une tresse de masse intégrée au câble électrique, à une borne de masse de l'appareil enregistreur pour éviter que le signal prélevé par l'électrode soit parasité par des rayonnements.

Cependant, il importe qu'au cours de la période pendant laquelle le patient doit porter les électrodes, les conducteurs reliés à celles-ci ne subissent pas de déplacements intempestifs générant des tractions s'exerçant sur les électrodes ; de même, il importe que les électrodes elles-mêmes ne soient pas soumises à des chocs ou à des efforts pouvant être provoqués par des frottements accidentels ; l'expérience a montré que malgré les précautions prises, il n'est pas toujours possible d'éviter de telles contraintes momentanées sur les fils et/ou les électrodes elles-mêmes.

Ces contraintes, chocs, frottements, etc., sont générateurs de tensions parasites qui constituent autant d'artefacts également enregistrés sur l'électrocardiogramme, et qui gênent considérablement la lecture et l'interprétation de celui-ci.

Pour éviter l'apparition de ces artefacts, de manière classique, on immobilise simplement les conducteurs reliés aux électrodes en les fixant sur la peau du patient à une certaine distance de l'électrode au moyen de fragments de ruban adhésif en créant de plus une bouche conférant au conducteur une certaine laxité entre le ruban adhésif et le connecteur ; cependant ce procédé est gênant pour le patient et pas totalement efficace, puisqu'il n'évite pas les inconvénients liés aux efforts s'exerçant directement sur l'électrode ou le connecteur.

On connaît également des dispositifs protecteurs d'électrode en forme de cuvette destinés à coiffer l'électrode, comportant une couronne périphérique munie d'un adhésif que l'on colle contre la peau du patient après avoir immobilisé le ou les fil(s) conducteur(s) entre la paroi adhésive de la couronne et la peau, là encore en créant une boucle.

Cette méthode, qui présente une meilleure efficacité, a cependant pour inconvénients de nécessiter un certain tour de main de la part du personnel appelé à mettre en place l'appareillage, d'augmenter la durée de l'opération de mise en place, d'augmenter l'encombrement de l'électrode, et d'impliquer un décollage parfois gênant pour le patient au moment du retrait de l'électrode.

L'invention a pour but de remédier à ces inconvénients, et concerne à cet effet un connecteur d'électrode, notamment d'électrode d'électrocardiogramme, munie d'une tête de connexion en matériau bon conducteur de l'électricité, connecteur du type comportant un organe de contact avec la tête de connexion, cet organe de contact étant également en matériau bon conducteur de l'électricité, un organe d'encliquetage du connecteur sur la tête de connexion, un capuchon de protection en matériau isolant électrique entourant au moins partiellement l'organe de contact, et un câble électrique comprenant au moins un conducteur électrique relié à l'organe de contact, connecteur d'électrode caractérisé en ce qu'il comporte également un dispositif de détection de contraintes génératrices d'artefacts comprenant un capteur de contrainte logé dans le capuchon, entre au moins une partie de l'organe de contact et au moins une zone interne du capuchon, et au moins un autre conducteur électrique, relié au capteur de contrainte.

L'invention concerne également un ensemble d'électrode notamment d'électrocardiogramme, du type comportant une électrode munie d'une tête de connexion reliée électriquement à une pastille imprégnée d'un gel conducteur de l'électricité, et un connecteur comportant un organe de contact avec la tête de connexion, cet organe de contact étant en matériau bon conducteur de l'électricité, un organe d'encliquetage du connecteur sur la tête de connexion, un capuchon de protection en matériau isolant électrique, entourant au moins partiellement l'organe de contact, et un câble électrique comprenant au moins un conducteur électrique relié à l'organe de contact, ensemble d'électrode caractérisé en ce que le connecteur comporte également un dispositif de détection de contraintes génératrices d'artefacts comprenant un capteur de contrainte logé dans le capuchon, entre au moins une partie de l'organe de contact et au moins une zone interne du capuchon, et au moins un autre conducteur électrique, relié au capteur de contrainte.

D'autres caractéristiques et avantages de l'invention ressortiront de la description qui va suivre donnée en référence aux dessins annexés, à titre d'exemple non limitatif, dessins dans lesquels :
- la figure 1 est une section schématique d'un ensemble d'électrode conventionnel, et
- la figure 2 est une section schématique d'un ensemble d'électrode selon l'invention.

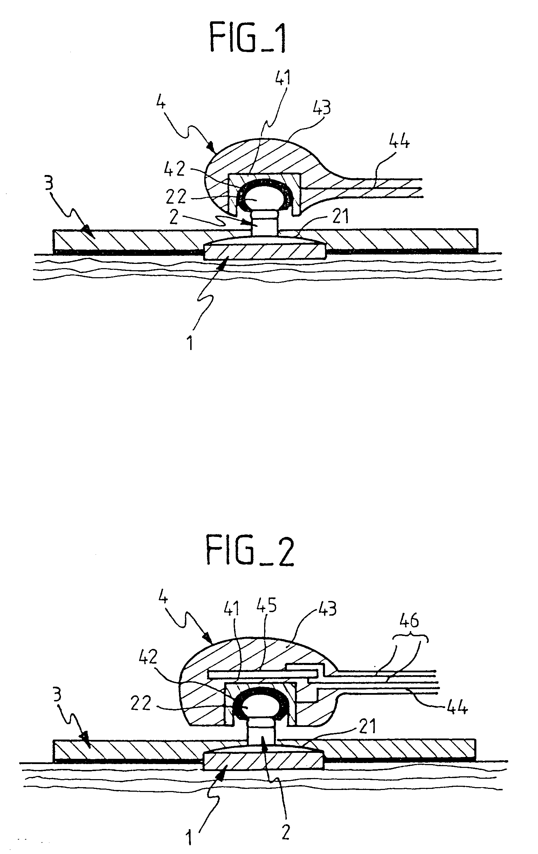

L'ensemble d'électrode représenté sur la figure 2, comme celui de la figure 1, est constitué d'une électrode d'électrocardiogramme et d'un connecteur, l'électrode comportant une pastille 1 en matériau mousse imprégné d'un gel bon conducteur de l'électricité, destinée à être appliquée contre la peau d'un patient, et un pion 2 également en matériau bon conducteur de l'électricité, par exemple métallique, muni d'une embase 21 accolée à la pastille 1 contre sa face opposée à la peau du patient, et d'une tête de connexion 22 munie d'une extrémité libre renflée de manière à permettre la solidarisation du connecteur par encliquetage. A la pastille 1, est solidarisé un disque 3 en matériau souple pouvant s'adapter au profil du corps du patient, dont une face est enduite d'un adhésif pour pouvoir être collée à l'épiderme de celui-ci, et muni d'un trou central pour pouvoir être enfilé autour de la tête de connexion et solidarisé à la pastille en mousse imprégnée ; ce disque peut être également en mousse.

Le connecteur 4 comporte un organe de contact 41 en matériau bon conducteur de l'électricité, par exemple métallique, destiné à venir en appui contre la tête de connexion 22 de l'électrode, au moins partiellement logé à l'intérieur d'un capuchon de protection 43 en matériau isolant électrique, un organe d'encliquetage amovible 42 logé dans l'organe de contact 41 pour maintenir celui-ci en contact avec la tête de connexion 22 en position d'encliquetage, et un capuchon de protection 43 en matériau isolant électrique à l'intérieur duquel est logé au moins partiellement l'organe de contact. L'organe d'encliquetage 42 est par exemple, de manière connue, une rondelle élastique fendue ou une agraffe élastique à deux branches. Un câble électrique comporte un conducteur 44 reliant l'organe de contact 41 à une borne d'entrée d'un appareil d'enregistrement d'électrocardiogramme non représenté.

Cependant, de plus, selon l'invention, le connecteur est muni d'un dispositif de détection de contraintes génératrices d'artefacts comprenant un capteur de contrainte 45 inséré entre au moins une partie de l'organe de contact 41 et le fond du capuchon de protection 43, et des conducteurs électriques 46 reliés à ce capteur de contrainte 45 pour transmettre tout signal ainsi capté à un montage adapté à soustraire ce signal capté du signal parasité par les artefacts issu de la pastille 1 via le pion 2, l'organe de contact 41 et le conducteur 44.

Avantageusement, le capteur de contrainte 45 peut comporter une plaquette à deux faces planes parallèles dont l'une est à proximité ou en contact d'une face plane correspondante de l'organe de contact 41.

Dans une forme particulière de réalisation de l'invention, c'est cette plaquette qui porte un élément sensible aux contraintes mécaniques, par exemple une jauge de contrainte à résistance.

Dans une autre forme particulière de l'invention, la plaquette constitue elle-même l'élément sensible aux contraintes, étant réalisée en un matériau piezo-électrique, par exemple une céramique piezo-électrique.

Les deux conducteurs électriques 46 reliés soit aux bornes respectives de l'élément sensible intégré à la plaquette ou porté par elle, soit aux deux faces de celle-ci si elle constitue elle-même cet élement sensible, sont de préférence deux conducteurs du câble électrique dont un conducteur 44 est relié à l'organe de contact 41 pour transmettre le signal provenant de la pastille 1. Dans certains cas, un seul conducteur 46 peut suffire, le signal de contrainte étant transmis par le conducteur 46 relié au capteur 45 et le conducteur 44 relié à l'organe de contact 41.

Avec cette disposition, toute contrainte mécanique exercée sur le câble électrique ou sur le connecteur 4 et transmise à l'électrode elle-même, génératrice d'artefacts, développe également une tension aux bornes du capteur, et cette tension, apparaissant à ces bornes, est transmise par les conducteurs 46 au montage soustracteur, appartenant par exemple à l'appareil d'enregistrement de la tension d'électrocardiogramme ; cet appareil permet donc de visualiser une tension d'électrocardiogramme dénuée d'artefacts, par suite de la soustraction du signal apparaissant aux bornes du capteur 45, à la tension transmise à l'appareil par le conducteur 44 relié à la tête d'électrode, ou encore par suite d'un autre traitement électronique. Le capuchon 43 peut également être muni d'un blindage métallique relié à une borne de masse par une gaine tressée entourant les conducteurs 44, 46. Dans l'hypothèse où il serait impossible de parvenir par soustraction à un résultat pouvant être considéré comme acceptable, il est également possible de prévoir de commander par le signal provenant du capteur, l'inhibition de l'analyse du traitement du signal en cours. La gestion de l'utilisation du signal provenant du capteur est avantageusement effectuée au moyen d'un montage à microprocesseur.

Bien entendu, l'invention n'est pas limitée aux formes de réalisation ci-dessus décrites et représentées, et on pourra en prévoir d'autres formes sans sortir de son cadre, par exemple l'invention peut être mise en oeuvre avec des électrodes autres qu'électrocardiographiques.

## Revendications

1. Connecteur d'électrode, notamment d'électrode d'électrocardiogramme, munie d'une tête de connexion (22) en matériau bon conducteur de l'électricité, ledit connecteur comportant un organe (41) de contact avec la tête de connexion (22), cet organe de contact (41) étant également en matériau bon conducteur de l'électricité, un organe d'encliquetage (42) du connecteur sur la tête de connexion, un capuchon de protection (43) en matériau isolant électrique entourant au moins partiellement l'organe de contact, et un câble électrique comprenant au moins un conducteur électrique (44) relié à l'organe de contact, connecteur d'électrode caractérisé en ce qu'il comporte également un dispositif de détection de contraintes génératrices d'artefacts comprenant un capteur de contrainte (45) logé dans le capuchon (43), entre au moins une partie de l'organe de contact (41) et au moins une zone interne du capuchon, et au moins un autre conducteur électrique (46), relié au capteur de contrainte (45).

2. Connecteur d'électrode selon la revendication 1, caractérisé en ce que le dispositif de détection de contraintes génératrices d'artefacts comprend un capteur de contrainte (45) sous la forme d'une plaquette portant un élément sensible aux contraintes.

3. Connecteur d'électrode selon la revendication 1, caractérisé en ce que le dispositif de détection de contraintes génératrices d'artefacts comprend un capteur de contrainte (45) sous la forme d'une plaquette constituant un élément sensible aux contraintes.

4. Connecteur d'électrode selon la revendication 1, caractérisé en ce que le capteur (45) est en matériau piezo-électrique.

5. Connecteur d'électrode selon la revendication 1, caractérisé en ce que le capteur (45) est en céramique piezo-électrique.

6. Connecteur d'électrode selon la revendication 1, caractérisé en ce que le capteur (45) porte une jauge de contrainte à résistance.

7. Connecteur d'électrode selon la revendication 1, caractérisé en ce qu'il comporte deux conducteurs électriques (46) reliés au capteur de contrainte (45).

8. Connecteur d'électrode selon la revendication 1, caractérisé en ce qu'il comporte au moins un conducteur électrique (46) relié au capteur de contrainte (45), associé au conducteur électrique (44) relié à l'organe de contact (41), pour constituer le câble électrique.

9. Ensemble d'électrode notamment d'électrocardiogramme, comportant une électrode (1, 2, 3) munie d'une tête de connexion (22) reliée électriquement à une pastille (1) imprégnée d'un gel conducteur de l'électricité, et un connecteur (4) comportant un organe (41) de contact avec la tête de connexion, cet organe de contact étant en matériau bon conducteur de l'électricité, un organe d'encliquetage (42) du connecteur sur la tête de connexion (22), un capuchon de protection (43) en matériau isolant électrique, entourant au moins partiellement l'organe de contact, et un câble électrique comprenant au moins un conducteur électrique (44) relié à l'organe de contact, ensemble d'électrode caractérisé en ce que le connecteur comporte également un dispositif de détection de contraintes génératrices d'artefacts comprenant un capteur de contrainte (45) logé dans le capuchon (43), entre au moins une partie de l'organe de contact (41) et au moins une zone interne du capuchon (43), et au moins un autre conducteur électrique (46), relié au capteur de contrainte (45).

## Claims

1. Electrode connector, in particular for an electrode of an electrocardiogram, equipped with a connecting head (22) made of a material with good electrical conduction, said connector comprising a member (41) for contact with the connecting head (22), this contact member (41) also being made of a material with good electrical conduction, a member (42) for locking the connector onto the connecting head, a protective cap (43) made of electrically insulating material at least partially surrounding the contact member, and an electric cable comprising at least one electrical conductor (44) connected to the contact member, said electrode connector being characterised in that it also comprises a device for detecting stresses generating artefacts, which comprises a stress sensor (45) disposed in the cap (43) between at least one portion of the contact member (41) and at least one internal zone of the cap, and at least one other electrical conductor (46) connected to the stress sensor (45).

2. Electrode connector according to Claim 1, characterised in that the device for detecting stresses generating artefacts comprises a stress sensor (45) in the form of a strip bearing an element which is sensitive to stresses.

3. Electrode connector according to Claim 1, characterised in that the device for detecting stresses generating artefacts comprises a stress sensor (45) in the form of a strip forming an element which is sensitive to stresses.

4. Electrode connector according to Claim 1, characterised in that the sensor (45) is made of a piezoelectrical material.

5. Electrode connector according to Claim 1, characterised in that the sensor (45) is made of a piezoelectrical ceramic.

6. Electrode connector according to Claim 1, characterised in that the sensor (45) bears a resistance stress gauge.

7. Electrode connector according to Claim 1, characterised in that it comprises two electrical conductors (46) connected to the stress sensor (45).

8. Electrode connector according to Claim 1, characterised in that it comprises at least one electrical conductor (46) connected to the stress sensor (45) which is allocated to the electrical conductor (44) connected to the contact member (41) to form the electric cable.

9. Electrode assembly, in particular for an electrocardiogram, comprising an electrode (1, 2, 3) equipped with a connecting head (22) connected electrically to a disc (1) impregnated with an electrically conductive gel, and a connector (4) comprising a member (41) for contact with the connecting head (22), this contact member (41) being made of a material with good electrical conduction, a member (42) for locking the connector onto the connecting head, a protective cap (43) made of electrically insulating material at least partially surrounding the contact member, and an electric cable comprising at least one electrical conductor (44) connected to the contact member, said electrode assembly being characterised in that the connector also comprises a device for detecting stresses generating artefacts, which comprises a stress sensor (45) disposed in the cap (43) between at least one portion of the contact member (41) and at least one internal zone of the cap, and at least one other electrical conductor (46) connected to the stress sensor (45).

## Patentansprüche

1. Elektrodenverbinder, insbesondere für eine Elektrokardiogramm-Elektrode, die mit einem Anschlußkopf (22) aus einem elektrisch gut leitendenden Material versehen ist, wobei der Verbinder aufweist: ein Kontaktorgan (41) zum Kontaktieren des Anschlußkopfes (22), das ebenfalls aus elektrisch gut leitendem Material besteht, ein Organ (42) zum Verrasten des Verbinders mit dem Anschlußkopf, eine Schutzhaube (43) aus elektrisch isolierendem Material, die das Kontaktorgan wenigstens teilweise umgibt, und ein elektrisches Kabel, das wenigstens einen elektrischen Leiter (44) aufweist, der mit dem Kontaktorgan verbunden ist, dadurch gekennzeichnet, daß der Elektrodenverbinder ferner eine Vorrichtung zum Feststellen von Fehler (Artefakte) verursachenden Belastungen aufweist, die einen Belastungsaufnehmer (45), der in der Haube (43) zwischen wenigstens einem Teil des Kontaktorgans (41) und wenigstens einer inneren Zone der Haube angeordnet ist, und wenigstens einen weiteren elektrischen Leiter (46), der am Belastungsaufnehmer (45) angeschlossen ist, aufweist.

2. Elektrodenverbinder nach Anspruch 1, dadurch gekennzeichnet, daß die Vorrichtung zum Feststellen von Fehler verursachenden Belastungen einen Belastungsaufnehmer (45) in Form einer Platte aufweist, die ein auf Belastungen empfindliches Element trägt.

3. Elektrodenverbinder nach Anspruch 1, dadurch gekennzeichnet, daß die Vorrichtung zum Feststellen von Fehler verursachenden Belastungen einen Belastungsaufnehmer (45) in Form einer Platte aufweist, die ein auf Belastungen empfindliches Element bildet.

4. Elektrodenverbinder nach Anspruch 1, dadurch gekennzeichnet, daß der Aufnehmer (45) aus einem piezoelektrischen Material besteht.

5. Elektrodenverbinder nach Anspruch 1, dadurch gekennzeichnet, daß der Aufnehmer (45) aus einer piezoelektrischen Keramik besteht.

6. Elektrodenverbinder nach Anspruch 1, dadurch gekennzeichnet, daß der Aufnehmer (45) einen Widerstands-Belastungsfühler trägt.

7. Elektrodenverbinder nach Anspruch 1, dadurch gekennzeichnet, daß er zwei elektrische Leiter (46) aufweist, die an dem Belastungsaufnehmer (45) angeschlossen sind.

8. Elektrodenverbinder nach Anspruch 1, dadurch gekennzeichnet, daß er wenigstens einen am Belastungsaufnehmer angeschlossenen elektrischen Leiter (46) aufweist, der mit dem am Kontaktorgan (41) angeschlossenen elektrischen Leiter (44) zur Bildung des elektrischen Kabels vereinigt ist.

9. Elektrodenanordnung, insbesondere Elektrokardiogramm-Elektrodenanordnung, mit einer Elektrode (1, 2, 3), die mit einem Anschlußkopf (22) versehen ist, der mit einem Plättchen (1) elektrisch verbunden ist, das mit einem elektrisch leitenden Gel imprägniert ist, und mit einem Verbinder (4), der ein Kontaktorgan (41) aus einem elektrisch gut leitenden Material zur Kontaktierung des Anschlußkopfes, ein Rastorgan (42) zum Einrasten des Verbinders auf dem Anschlußkopf (22), eine Schutzhaube (43) aus elektrisch isolierendem Material, die das Kontaktorgan wenigstens teilweise umgibt, und ein elektrisches Kabel aufweist, das wenigstens einen elektrischen Leiter (44) enthält, der mit dem Kontaktorgan verbunden ist, dadurch gekennzeichnet, daß der Verbinder ferner eine Vorrichtung zum Feststellen von Fehler (Artefakte) verursachenden Belastungen mit einem Belastungsaufnehmer (45), der in der Haube (43) zwischen wenigstens einem Teil des Kontaktorgans (41) und wenigstens einer inneren Zone der Haube (43) angeordnet ist, und wenigstens einen äußeren elektrischen Leiter (46), der mit dem Belastungsaufnehmer (45) verbunden ist, aufweist.
